# EUROPÄISCHE PATENTSCHRIFT

(11) **EP 1 531 154 B1**
(45) Veröffentlichungstag und Bekanntmachung des Hinweises auf die Patenterteilung: **14.04.2010**
(21) Anmeldenummer: 04025731.3
(22) Anmeldetag: 29.10.2004
(51) Int. Cl.: C07F 5/02, C07D 409/14

(54) **Verfahren zur Herstellung linearer organischer Thiophen-Phenylen-Oligomere**
Process for the preparation of linear organic thiophene/phenylene oligomers
Procédé pour la préparation des oligomères organiques linéaires de thiophène et de phénylène

(30) Priorität: 12.11.2003 DE 10353094
(43) Veröffentlichungstag der Anmeldung: 18.05.2005
(60) Teilanmeldung: 07114437.2
(73) Patentinhaber: H.C. Starck Clevios GmbH, 38642 Goslar (DE)
(72) Erfinder: Kirchmeyer, Stephan, Dr., 51375 Leverkusen (DE); Ponomarenko, Sergei, Dr., 107589 Moskau (DE)
(74) Vertreter: Herzog, Martin

(56) Entgegenhaltungen:
- EP-A- 0 439 627
- WO-A-2004/003108
- US-A1- 2002 114 973
- HONG X M ET AL: "THIOPHENE-PHENYLENE AND THIOPHENE-THIAZOLE OLIGOMERIC SEMICONDUCTORS WITH HIGH FIELD-EFFECT TRANSISTOR ON/OFF RATIOS" CHEMISTRY OF MATERIALS, AMERICAN CHEMICAL SOCIETY, WASHINGTON, US, Bd. 13, Nr. 12, 2001, Seiten 4686-4691, XP001150369 ISSN: 0897-4756
- MELUCCI, M. ET AL: "Solution-Phase Microwave-Assisted Synthesis of Unsubstituted and Modified .alpha.-Quinque- and Sexithiophenes Solution-Phase Microwave-Assisted Synthesis of Unsubstituted and Modified .alpha.-Quinque- and Sexithiophenes" JOURNAL OF ORGANIC CHEMISTRY , 69(14), 4821-4828 CODEN: JOCEAH; ISSN: 0022-3263, 11. August 2002 (2002-08-11), XP002319830
- HENZE, OLIVER ET AL: "The synthesis and characterization of amphiphilic .alpha.,.alpha.-linked sexithiophenes with substituents at the terminal .alpha.-positions The synthesis and characterization of amphiphilic .alpha.,.alpha.-linked sexithiophenes with substituents at the terminal .alpha.-positions" JOURNAL OF MATERIALS CHEMISTRY , 13(6), 1269-1273 CODEN: JMACEP; ISSN: 0959-9428, 22. April 2003 (2003-04-22), XP002319831
- CHRISTOPHERSEN, CLAUS ET AL: "Synthesis of 2,3-Substituted Thienylboronic Acids and Esters Synthesis of 2,3-Substituted Thienylboronic Acids and Esters" JOURNAL OF ORGANIC CHEMISTRY , 68(24), 9513-9516 CODEN: JOCEAH; ISSN: 0022-3263, 31. Oktober 2003 (2003-10-31), XP002330210
- PATENT ABSTRACTS OF JAPAN Bd. 016, Nr. 465 (E-1270), 28. September 1992 (1992-09-28) & JP 04 164370 A (IDEMITSU KOSAN CO LTD), 10. Juni 1992 (1992-06-10)
- SATO T ET AL: "PHOTOPHYSICAL PROPERTIES OF BIS(2,2'-BITHIOPHENE-5-YL)BENZENES" JOURNAL OF THE CHEMICAL SOCIETY. FARADAY TRANSACTIONS, ROYAL SOCIETY OF CHEMISTRY, CAMBRIDGE, GB, Bd. 94, Nr. 16, 21. August 1998 (1998-08-21), Seiten 2355-2360, XP000776073 ISSN: 0956-5000
- PORZIO W ET AL: "Organic FET devices: structure-property relationship in evaporated films of three fluorenone derivatives" SYNTHETIC METALS, ELSEVIER SEQUOIA, LAUSANNE, CH, Bd. 146, Nr. 3, 3. November 2004 (2004-11-03), Seiten 259-263, XP004629438 ISSN: 0379-6779
- TIRAPATTUR S ET AL: "Spectroscopic Study of Intermolecular Interactions in Various Oligofluorenes: Precursors of Light-Emitting Polymers" JOURNAL OF PHYSICAL CHEMISTRY, AMERICAN CHEMICAL SOCIETY, US, Bd. 106, 2002, Seiten 8959-8966, XP002321534 ISSN: 0022-3654

## Beschreibung

Die Erfindung betrifft ein neues Verfahren zur Herstellung linearer organischer Thiophen-Phenylen-Oligomere.

Das Feld molekularer Elektronik hat sich in den letzten 15 Jahren mit der Entdeckung organischer leitender und halbleitender Verbindungen rapide entwickelt. In dieser Zeit wurden eine Vielzahl von Verbindungen gefunden, die halbleitende oder elektrooptische Eigenschaften aufweisen. Wichtige Vertreter halbleitender Verbindungen sind neben Oligothiophenen auch beispielsweise Thiophen-Phenylen-Cooligomere. Solche Verbindungen sind derzeit für Anwendungsgebiete wie organische Feld-Effekt-Transistoren (OFET's), organische Lumineszenzdioden (OLED's), Sensoren und photovoltaische Elemente von großem Interesse.

Die Herstellung von Thiophen-Phenylen-Cooligomere, im Folgenden auch als Thiophen-Phenylen-Oligomere bezeichnet, kann mittels unterschiedlicher, dem Fachmann bekannten Kupplungsreaktionen erfolgen. Dass die Eignung der Endprodukte für den Einsatz in der Halbleitertechnik wesentlich von deren Reinheit abhängt und die nachteiligen Auswirkungen verunreinigter Halbleiter auf die Eigenschaften, wie beispielsweise ein schlechtes "On/Off Ratio" in OFETs, der daraus hergestellten elektronischen Anwendungen sind allgemein bekannt (Handbook of Oligo- and Polythiophenes, ed. by D. Fischou, Wiley-VCH, Weinheim, S. 469-471).

Hotta et al. beschreibt eine Reihe von Phenylen-Oligothiophenen und deren Herstellung über Grignard-Reaktionen oder Suzuki-Kupplung, wobei jedoch so stark verunreinigte Produkte gebildet werden, dass nach Aufreinigung hohe Ausbeuteverluste zu verzeichnen sind (J. Heterocyclic Chem. 2000, 37, 281-286; J. Heterocyclic Chem. 2000, 37, 25-29, Synt. Met. 1999, 101, 551-552). Die Suzuki-Kupplung erfolgt in allen Fällen zwischen Aryl-Boronsäuren und Thienylhalogeniden. Die Eignung von Oligothiophenen mit Phenyleinheiten als Endgruppen als lichtemittierende Schichtmaterialien in OLEDs wurde von Hotta et al. in Synt. Met. 1999, 106, 39-40 beschrieben.

Katz et al. beschrieb kürzlich weitere Thiophen-Phenylen-Oligomere und deren Eignung als oligomere Halbleiter für OFET's. Unglücklicherweise konnte auch hier das am besten geeignete 1,4-Bis(5-hexyl-2,2'-bithien-5-yl)phenyl durch Kupplung von zinnorganischen Thiophen-Verbindungen mit Dihalogenarylverbindungen lediglich in einer extrem niedrigen Ausbeute von 10 % hergestellt werden, so dass diese Methode zur Herstellung größerer Mengen an Oligomeren nicht geeignet ist (chem. Mater. 2001, 13, 4686-4691). Katz et al. beschreibt zudem die Synthese von 1,4-Bis(2,2'-bithien-5-yl)phenyl und 1,4-Bis(4-hexyl-2,2'-bithien-5-yl)phenyl über Suzuki-Kupplung entsprechender Thiophen-Phenylen-Thiophen-Dihalogenide mit Monothiophen-Boronsäuren. Nachteilig bei dieser Methode ist jedoch, dass eine zentrale Thiophen-Phenylen-Thiophen-Dihalogenideinheit durch aufwändige Kupplungsreaktionen hergestellt werden muss. Zudem wird Thiophenboronsäuren eine geringe Stabilität zugeschrieben (Gronowitz et.al., Heterocycles 1990, 30, 645-658; Lehn et al., Eur. Chem. J. 1996, 2, 1399-14.06). Beides resultiert in hohen Ausbeuteverlusten und/oder einer größeren Menge von Verunreinigungen.

In US-A 2002/0114973 werden weitere Thiophen-Phenylen-Oligomere beschrieben, wobei jedoch weiterhin der Nachteil besteht, dass mit zunehmender Kettenlänge zunehmend viele Kupplungsschritte zum Aufbau des Moleküls erforderlich sind, was weiterhin das Problem des hohen Ausbeuteverlustes und größerer Mengen an Verunreinigungen, die über aufwändige und verlustreiche Aufreinigung beseitigt werden müssen, bestehen lässt. Auch in US-A 2002/0114973 wird die Herstellung der Thiophen-Phenylen-Oligomere mittels Grignard-Reaktionen und/oder Suzuki-Kupplung unter Einsatz von Thiophenboronsäure beschrieben.

Es bestand daher weiterhin Bedarf an einem Verfahren zur Herstellung halbleitender organischer Thiophen-Phenylen-Oligomere, bei denen die Endprodukte über möglichst wenige Zwischenprodukte und in hoher Reinheit und guter Ausbeute erhalten werden. Vorteilhafterweise sollte sich ein solches Verfahren zur Herstellung solcher Oligomeren unabhängig von deren Kettenlänge anwenden lassen.

Die Aufgabe der vorliegenden Erfindung bestand somit darin, ein solches Verfahren bereitzustellen.

Überraschend wurde nun gefunden, dass eine Suzuki-Kupplung in der als bororganische Verbindung ein Thiophenpinakolinboronsäureester mit mehr als einer Thiopheneinheit eingesetzt wird, diese Anforderungen erfüllt.

Gegenstand der vorliegenden Erfindung ist daher ein Verfahren zur Herstellung von Verbindungen der allgemeinen Formel (I), 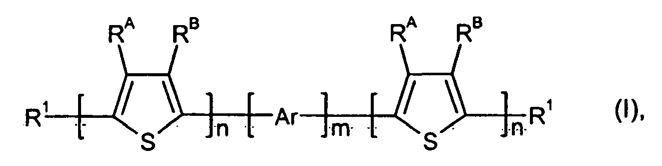 insbesondere nach dem Prinzip der Suzuki-Kupplung, worin
- m: für eine ganze Zahl von 1 bis 5, bevorzugt für 1, 2 oder 3, besonders bevorzugt für 1 steht,
- Ar: für gegebenenfalls substituiertes 1,4-Phenylen und/oder 2,7-Fluorenylen steht,
- R¹: für H oder eine gegebenenfalls substituierte lineare oder verzweigte C₁-C₂₀-Alkylgruppe, bevorzugt eine C₁-C₁₂-Alkylgruppe, besonders bevorzugt eine Ethyl-, n-Butyl, n-Hexyl-, n-Octyl, n-Decyl oder n-Dodecylgruppe, oder eine gegebenenfalls durch ein(e) oder mehrere O- oder S-Atome, Silylen-, Phosphonoyl- oder Phosphorylgruppen unterbrochene lineare C₁-C₂₀-Alkylgruppe, bevorzugt C₁-C₁₂-Alkylgruppe, oder eine C₃-C₂₀-Alkenylgruppe, bevorzugt eine C₁-C₁₂-Alkenylgruppe, besonders bevorzugt eine Allyl-, Butenyl-, Pentenyl-, Hexenyl-, Heptenyl-, Octenyl-, Decenyl-, Undecenyl- oder Dodecenyl-Gruppesteht ,
- R^{A}, R^{B}: unabhängig voneinander jeweils für H oder eine gegebenenfalls substituierte und/oder gegebenenfalls mit bis zu 10 Sauerstoffatomen unterbrochene lineare oder verzweigte C₁-C₂₀-Alkylgruppe, bevorzugt für eine C₁-C₁₂-Alkylgruppe, besonders bevorzugt für eine Methyl-, Ethyl-, Propyl-, Butyl-, Pentyl- oder Hexylgruppe, eine gegebenenfalls substituierte C₁-C₂₀-Alkoxygruppe, bevorzugt für eine C₁-C₆-Alkoxygruppe, besonders bevorzugt für eine Methoxygruppe, oder gemeinsam für eine gegebenenfalls substituierte C₁-C₆-Dioxyalkylengruppe, bevorzugt für eine 3,4-Alkylendioxygruppe mit bis zu 6 Kohlenstoffatomen in der Alkylengruppe, beispielsweise eine 3,4-Ethylendioxygruppe oder 3,4-Propylendioxygruppe, stehen und
- n: für 2, 3 oder 4 steht,
dadurch gekennzeichnet, dass eine Verbindung der allgemeinen Formel (II), 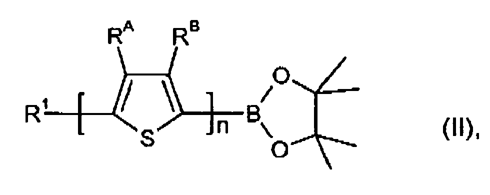 worin
n, R¹, R^{A} und R^{B} die oben für die allgemeine Formel (I) genannte Bedeutung haben.
mit einer Verbindung der allgemeinen Formel (III), 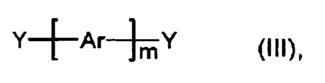 worin
- m und Ar: die für die allgemeine Formel (I) genannte Bedeutung haben und worin
- Y: für einen Rest Cl, Br, I oder O-SO₂-R^{C} steht, wobei R^{C} für einen Phenyl-, Tolyl-, Methyl- oder Trifluormethylrest steht,
gegebenenfalls in Anwesenheit von Katalysatoren, gegebenenfalls in Lösung und gegebenenfalls bei erhöhter Temperatur umgesetzt wird.

Dabei können die n Thiopheneinheiten in den Verbindungen der allgemeinen Formeln(I) oder (II) gleich oder verschieden sein, d.h. die Substituenten R^{A} können in den einzelnen Thiopheneinheiten gleich oder verschieden sein und die Substituenten R^{B} können in den einzelnen Thiopheneinheiten gleich oder verschieden sein.

In einer bevorzugten Ausführungsform der Erfindung stehen R^{A} und R^{B} für H.

Für den Fall, dass R¹ für eine C₃-C₂₀-Alkenylgruppe steht, ist die Doppelbindung der C₃-C₂₀-Alkenylgruppe bevorzugt endständig (vgl. z.B. Formel II-a in Beispiel 1).

In der allgemeinen Formeln (I) und (II) und in weiteren Formeln dieser Anmeldung ist die Strukturformel des Pinakolinboronsäurerestes 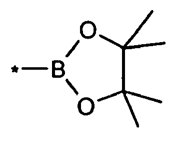 als vereinfachte Form der Strukturformel 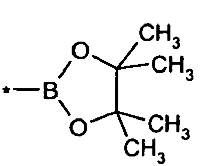 zu verstehen.

Als Substituenten für den Rest Ar kommen beispielsweise lineare oder verzweigte C₁-C₂₀-Alkylreste, bevorzugt C₁-C₁₂-Alkylreste, oder durch ein(e) oder mehrere O-Atome unterbrochene lineare C₁-C₂₀-Alkylreste in Frage. An den 2,7-Fluorenyleneinheiten sitzen (einer oder mehrere) gegebenenfalls vorhandene Substituenten bevorzugt an der 9-Position. Besonderes bevorzugte Substituenten 1,4-Phenylen-Ringe sind Methyl-, Ethyl-, Propyl-, Butyl-, Pentyl- oder Hexyl-Gruppen. Weitere besonderes bevorzugte Substituenten für Phenyl-Gruppen sind Methoxy-, Ethoxy-, Propoxy-, Buthoxy-, Pentoxy-, Hexyloxy- oder Heptyloxy-Gruppen. Ganz besonderes bevorzugter Substituent ist Wasserstoff.

Bevorzugt ist das neue Verfahren zur Herstellung von Verbindungen der allgemeinen Formel (I-a) 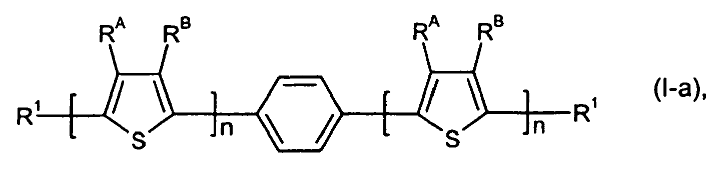 worin
n, R¹, R^{A} und R^{B} die oben für die allgemeine Formel (I) genannte Bedeutung haben,
wobei eine Verbindung der allgemeinen Formel (II) mit einer Verbindung der allgemeinen Formel (III-a) 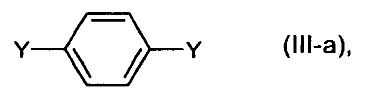 worin
- Y: die oben für die allgemeine Formel (III) genannte Bedeutung hat,
umgesetzt wird.

Beispielhaft für bevorzugt hergestellte Verbindungen der allgemeinen Formel (I-a) sind 5-Alkyl-5'-[4-(5'-alkyl-2,2'-bithien-5-yl)phenyl]-2,2"-bithiophen und 5-Alkyl-5"-[4-(5"-alkyl-2,2':5',2"-terthien-5-yl)phenyl]-2,2':5',2"-terthiophen, insbesondere 5-Decyl-5"-[4-(5"-decyl-2,2':5',2"-terthien-5-yl)phenyl]-2,2':5',2"-terthiophen, 5-Hexyl-5"-[4-(5"-hexyl-2,2':5',2"-terthien-5-yl)phenyl]-2,2':5',2"-terthiophen, 5-Ethyl-5"-[4-(5"-Ethyl-2,2':5',2"-terthien-5-yl)phenyl]-2,2':5',2"-terthiophen, 5-Decyl-5'-[4-(5'-Decyl-2,2'-bithien-5-yl)phenyl]-2,2"-bithiophen, 5-Hexyl-5'-[4-(5'-hexyl-2,2'-bithien-5-yl)phenyl]-2,2"-bithiophen und 5-Etyl-5'-[4-(5'-ethyl-2,2'-bithien-5-yl)phenyl]-2,2"-bithiophen, genannt.

Solche Verbindungen sind bereits generell in US 6,355,365 B1 und US 2002/0114973 A1 beschrieben. Jedoch können sie nach dem neuen Verfahren erstmals in besonderer Reinheit, d.h. beispielsweise ohne Verunreinigungen durch strukturelle Isomere, z.B. durch 3,5- bzw. 2,4-verknüpfte Thiopheneinheiten, oder homologe Verbindungen, und nach einfacher einstufiger Umsetzung von Verbindungen der allgemeinen Formel (II) mit Aryldihalogeniden erhalten werden. In US 2002/0114973 sind zur Herstellung aufwändige mehrstufige Verfahren beschrieben, die in verminderter Ausbeute und/oder Reinheit der Zielverbindungen resultieren.

Bevorzugt wird das erfindungsgemäße Verfahren in einer Variante der Suzuki-Kupplung durchgeführt. Die Suzuki-Kupplung, d.h. die Umsetzung von Arylhalogeniden und bororganischen Verbindungen ist z.B. in Suzuki et al., Chem. Rev. 1995, 95, 2457-2483 beschrieben. In einer bevorzugten Ausführungsform wird das neue Verfahren gemäß einer Variante dieser Suzuki-Kupplung durchgeführt, wobei Aryl- bzw. Heteroarylhalogenide und Verbindungen der allgemeinen Formel (I) gegebenenfalls in Anwesenheit wenigstens einer Base und/oder wenigstens eines Katalysators, der ein Metall der VIII. Nebengruppe des Periodensystems der Elemente, im Folgenden kurz als Metall der VIII. Nebengruppe bezeichnet, enthält, umgesetzt werden.

Die besonders bevorzugte Ausführungsform des Verfahrens (Suzuki-Kupplung) wird bei einer Temperatur von +20°C bis +200°C, bevorzugt von +40°C bis +150°C, besonderes bevorzugt von +80°C bis +130°C, in einem organischen Lösungsmittel oder Lösungsmittelgemisch durchgeführt.

Als Katalysatoren, kommen prinzipiell alle Verbindungen in Frage, die ein Metall der VIII. Nebengruppe des Periodensystems der Elemente, bevorzugt Pd, Ni oder Pt, besonders bevorzugt Pd, enthalten. Der oder die Katalysator(en) werden bevorzugt in Mengen von 0,05 Gew.-% bis 10 Gew.-%, besonders bevorzugt von 0,5 Gew.-% bis 5 Gew.-%, bezogen auf das Gesamtgewicht der zu kuppelnden Verbindungen. verwendet. Besonders geeignete Katalysatoren sind Komplexverbindungen von Metallen der VIII. Nebengruppe, insbesondere Komplexe des Palladium(0), die an Luft stabil sind, Pd-Komplexe die sich leicht mit Organometallreagenzien (z.B. Lithiumalkylverbindungen oder magnesiumorganische Verbindungen) oder Phosphinen zu Palladium(0)-Komplexen reduzieren lassen, oder Palladium(2)-Komplexen gegebenenfalls unter Zusatz von PPh₃ oder anderen Phosphinen. Beispielsweise können PdCl₂(PPh₃)₂, PdBr₂(PPh₃)₂ oder Pd(OAc)₂ oder Mischungen aus diesen Verbindungen unter Zusatz von PPh₃ eingesetzt werden. Bevorzugt wird Pd(PPh₃)₄ ohne oder unter Zusatz von Phosphinen, in einer bevorzugten Ausführungsform ohne Zusatz von Phosphinen, eingesetzt, welches in preiswerter Form zur Verfügung steht. Als Phosphine werden bevorzugt PPh₃, PEtPh₂, PMePh₂, PEt₂Ph oder PEt₃, besonders bevorzugt PPh₃, eingesetzt.

Es ist jedoch auch möglich als Katalysatoren Palladiumverbindungen ohne Phosphinzusatz einzusetzen, beispielsweise Pd(OAc)₂.

Als Base können beispielsweise Hydroxide, z.B. NaOH, KOH, LiOH, Ba(OH)₂, Ca(OH)₂, Alkoxide, z.B. NaOEt, KOEt, LiOEt, NaOMe, KOMe, LiOMe, Alkalimetallsalze von Carbonsäuren, z.B. Natrium-, Kalium- oder Lithiumcarbonat, -hydrogencarbonat, -acetat, -citrat, -acetylacetonat, -glycinat, oder andere Carbonate, z.B. Cs₂CO₃ oder Tl₂CO₃, Phosphate, z.B. Natriumphosphat, Kaliumphosphat oder Lithiumphosphat, oder Mischungen aus diesen eingesetzt werden. Bevorzugt wird Natriumcarbonat eingesetzt. Die Basen können als Lösungen in Wasser oder als Suspensionen in organischen Lösungsmitteln, wie Toluol, Dioxan oder DMF, eingesetzt werden. Bevorzugt sind Lösungen in Wasser, da die erhaltenen Produkte aufgrund ihrer geringen Löslichkeit in Wasser so vom Reaktionsgemisch leicht abgetrennt werden können.

Es ist auch möglich weitere Salze, wie beispielsweise LiCl oder LiBr, als Hilfsmittel einzusetzen.

Als organische Lösungsmittel kommen grundsätzlich alle Lösungsmittel oder Lösungsmittelgemische in Frage, welche mit den Pinakolinboronsäureestem der allgemeinen Formel (II) nicht reagieren. Dies sind in der Regel Verbindungen, welche keine Halogenatome oder keine gegenüber Thiophen-Pinakolinboronsäureestem reaktive Wasserstoffatome aufweisen. Geeignete Lösungsmittel sind beispielsweise Alkane wie Pentan, Hexan und Heptan, Aromaten wie Benzol, Toluol und Xylole, Ethergruppen enthaltende Verbindungen wie Dioxan, Dimethoxyethan und Tetrahydrofuran und polare Lösungsmittel wie Dimethylformamid oder Dimethylsulfoxid. Bevorzugt werden im neuen Verfahren Aromaten als Lösungsmittel eingesetzt. Ganz besonders bevorzugt ist Toluol. Es ist auch möglich, als Lösungsmittel Mischungen aus zwei oder mehreren dieser Lösungsmittel einzusetzen.

Die Aufarbeitung der Reaktionsmischung erfolgt nach an sich bekannten Verfahren, z.B. durch Verdünnen, Fällen, Filtration, Extraktion, Waschen, Rekristallisation aus geeigneten Lösungsmitteln, Chromatographie und/oder Sublimation. Beispielsweise kann eine Aufarbeitung in der Weise erfolgen, dass die Reaktionsmischung nach vollständiger Reaktion in ein Gemisch aus saurem (Eis-)Wasser, z.B. hergestellt aus 1 molarer Salzsäure, und Toluol gegossen, die organische Phase abgetrennt, mit Wasser gewaschen, das als Feststoff enthaltene Produkt abfiltriert, mit Toluol gewaschen und anschließend im Vakuum getrocknet wird. Die Verbindungen der allgemeinen Formel (I) können bereits ohne weitere anschließende Reinigungsprozesse in hoher Qualität und Reinheit erhalten werden und sind halbleitend. Es ist jedoch möglich, diese Produkte nach bekannten Verfahren, z.B. durch Rekristallisation, Chromatographie oder Sublimation weiter zu reinigen.

Die in diesem Verfahren eingesetzten Aryldihalogenverbindungen der allgemeinen Formel (III) können nach an sich bekannten Verfahren hergestellt werden. Die Herstellung der Pinakolinboronsäureester der allgemeinen Formel (II) wird beispielsweise in Feast et al, J. Mater. Chem. 2003, 13, 1269-1273 beschrieben, wobei allerdings Zinnorganische Verbindungen als Ausgangsverbindungen eingesetzt werden. Aufgrund ihrer gesundheitlichen Bedenklichkeit wäre es jedoch vorteilhaft, den Einsatz solcher zinnorganischen Verbindungen zu vermeiden.

Alternativ können die Verbindungen der allgemeinen Formel (II) nach verschiedenen Verfahren hergestellt werden, die dem Fachmann grundsätzlich bekannt sind. Beispielsweise ist es möglich, die Verbindungen der allgemeinen Formel (II) durch Umsetzung von Arylhalogeniden und Bis-(pinakolato)diboran durch metallkatalysierte Kupplung herzustellen, wie sie beispielsweise in WO-A 01/29051 A1 und Tetrahedron Lett. 2002, S. 5649 beschrieben ist. Weiterhin ist es möglich die Verbindungen der allgemeinen Formel (II) durch Kupplung von Oligothiophenhalogeniden mit Pinakolboran herzustellen. (J. Org. Chem. 1997, B.62, S. 6458; J. Organomet. Chem. 2001, B. 640, S. 197; Chem. Commun. 2002, S. 1566). Diese Reaktionen führen jedoch zu Produkten, die Diaryle als Nebenprodukte enthalten, welche von den Pinakolinboronsäureestern der allgemeinen Formel (II) und besonders von den erfindungsgemäß herzustellenden Thiophen-Phenylen-Oligomeren der allgemeinen Formel (I) schlecht zu trennen sind.

Die Verbindungen der allgemeinen Formel (II) lassen sich nach bekannten Methoden der Aufarbeitung z.B. durch einfachen Abfiltrieren aus der Reaktionslösung isolieren und gegebenenfalls weiter reinigen. Als Reinigungsmethoden sind beispielsweise geeignet Rekristallisation, Sublimation oder Chromatographie. Die Verbindungen der allgemeinen Formel (II) können entweder in isolierter Form oder direkt in der Reaktionslösung ("in situ") hergestellt und ohne weitere Aufarbeitung eingesetzt werden.

Beispielhaft für Verbindungen der allgemeinen Formel (II) seien 4,4,5,5-Tetramethyl-2-[5'-(10-undecenyl)-2,2'-bithien-5-yl]-1,3,2-dioxaborolan (II-a), 2-(5"-Decyl-2,2':5',2"-terthien-5-yl)-4,4,5,5-tetramethyl-1,3,2-dioxaborolan (II-b), 4,4,5,5-Tetramethyl-2-[5'-hexyl-2,2'-bithien-5-yl]-1,3,2-dioxaborolan (II-c), 2-(5"-Hexyl-2,2':5',2"-terthien-5-yl)-4,4,5,5-tetramethyl-1,3,2-dioxaborolan (II-d) genannt:

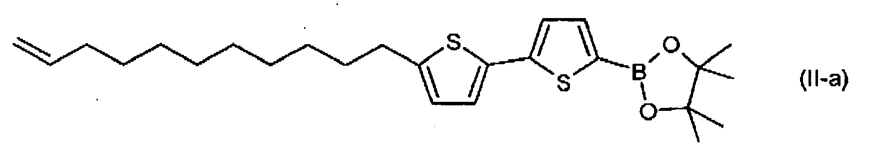

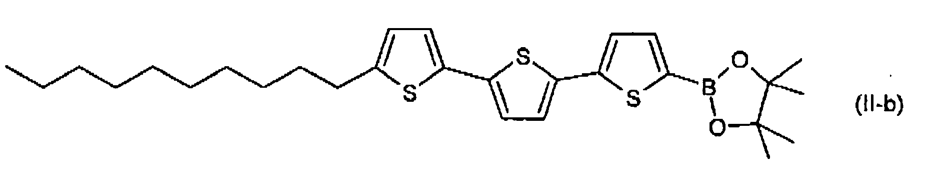

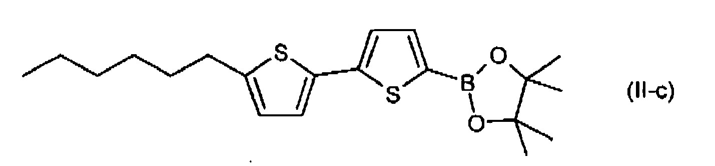

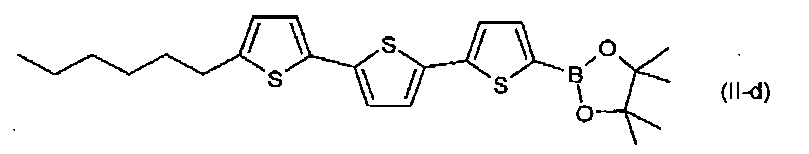

Bevorzugt werden die Verbindungen der allgemeinen Formel (II) in einer Reinheit von mindestens 90 %, besonders bevorzugt von mindestens 95 % erhalten.

Die Verbindungen der allgemeinen Formel (II) zeichnen sich durch besondere thermische Stabilität und Hydrolysebeständigkeit aus. Sie können nach dem erfindungsgemäßen Verfahren auf einfachem Wege hergestellt und isoliert werden und sind in hoher Reinheit zugänglich, so dass sie hervorragend als Precursor-Moleküle zur erfindungsgemäßen Herstellung halbleitender Thiophen-Phenylen-Oligomeren der allgemeinen Formel (I) geeignet sind. Überraschend tritt dabei keine Deborierung als Nebenreaktion auf und die erfindungsgemäße Herstellung der Thiophen-Phenylen-Oligomere lässt sich auch unter Bedingungen, wie beispielsweise unter Anwesenheit protischer Lösungsmittel, z.B. Toluol-Wasser-Gemischen, Natriumcarbonat als Base und unter Katalyse mit Tetrakis(triphenylphosphin)palladium durchführen, obwohl bekannt ist, dass Thiophenboronsäuren, wie z.B. 2-Thiophenboronsäure unter diesen Bedingungen schnell zu Borsäure und Thiophen hydrolysieren können und dass dann überwiegend Nebenprodukte erhalten werden (Chemica Scripta, 1984, 23, 120).

Mit dem erfindungsgemäßen Verfahren können somit erstmals ohne aufwändige Reinigungsverfahren Thiophen-Phenylen-Oligomere der allgemeinen Formel (I) mit nur sehr geringen Mengen an Verunreinigungen hergestellt werden. Insbesondere werden die erfindungsgemäß hergestellten Verbindungen der allgemeinen Formel (I) weitgehend frei von schwer abtrennbaren homologen Oligomeren mit höherem oder niedrigerem Molekulargewicht erhalten, so dass eine aufwändige Aufreinigung schwer trennbarer Gemische entfällt.

Bevorzugt werden die Verbindungen der allgemeinen Formel (I) in einer Reinheit von mindestens 95 %, besonders bevorzugt mindestens 99 % erhalten.

### Beispiele

5-(10-Undecenyl)-2,2'-bithiophen, 5-Decyl-2,2':5',2"-terthiophen, 5-Bromo-5'-Ethyl-2,2'-bithiophen und 5-Bromo-5"-ethyl-2,2':5',2"-terthiophen wurden nach bekannten Verfahren hergestellt (Synthesis, 1993, S.1099; Chem. Mater., 1993, Band 5, S. 430; J. Mater. Chem. 2003, Band 13, S. 197).

Alle Reaktionsgefäße wurden vor Gebrauch nach üblicher Schutzgastechnik ausgeheizt und mit Stickstoff geflutet.

### Beispiel 1: Herstellung von 4,4,5,5-Tetramethyl-2-[5'-(10-undecenyl)-2,2'-bithien-5-yl]-1,3,2-dioxaborolan (II-a)

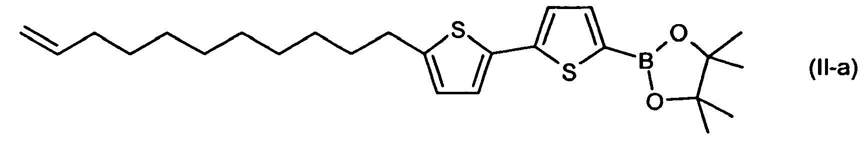

70 ml wasserfreies Tetrahydrofuran (THF) wurden mittels Trockeneis/Aceton auf -74°C heruntergekühlt. Hierzu wurden mit einer Spritze 5.6 ml einer 2.5 M Butyllithiumlösung in Hexan zugetropft. Anschließend wurde ein homogenes Gemisch aus 5-(10-Undecenyl)-2,2'-bithiophen (4.46 g, 14 mmol) in 120 ml wasserfreiem THF zugetropft und 30 min bei -74° nachgerührt. Das Kältebad wurde entfernt, so dass die Temperatur ansteigt. Bei ca. 0°C wurde der Reaktionsansatz wieder heruntergekühlt und bei - 74°C 2-Isopropoxy-4,4,5,5-tetramethyl-1,3,2-dioxaborolan (3.3 ml, 16 mmol) über einer Spritze zugegeben. Es wurde 30 min bei -74°C nachgerührt, das Kühlbad erneut entfernt und die Temperatur auf 20 °C ansteigen gelassen. Das Reaktionsgemisch wurde in 200 ml eisgekühltes Wasser - gemischt mit 15 ml 1 M HCl - gegeben und mit 500 ml Diethylether extrahiert. Die Etherphase wurde abgetrennt, mit Wasser gewaschen, über Na₂SO₄ getrocknet, filtriert und das Lösungsmittel vollständig am Rotationsverdampfer entfernt. Ausbeute: 6.03 g (97 % der Theorie) dunkelblaues, kristallines Produkt (II-a).

### Analysendaten:

GC MS Analyse: M^{•+} 99%, m/e = 444.
¹H NMR (CDCl₃, TMS/ppm): 1.22-1.45 (overlapped peaks with a max at 1.283, 14H), 1.345 (s, 12 H), 1.672 (m, J=7.5 Hz, M = 5, 2H), 2.037 (q, J=7.2 Hz, 2H), 2.781 (t, J=7.3 Hz, 2H), 4.928 (d, J=10.3 Hz, 1H), 4.991 (d, J=17.1 Hz, 1H), 5.811 (m, 1H), 6.676 (d, J=3.4 Hz, 1H), 7.037 (d, J=3.9 Hz, 1H), 7.152 (d, J=3.9 Hz, 1H), 7.496 (d, J=3.4 Hz, 1H).

### Beispiel 2: Herstellung von 2-(5"-Decyl-2,2':5',2"-terthien-5-yl)-4,4,5,5-tetramethyl-1,3,2-dioxaborolan (II-b)

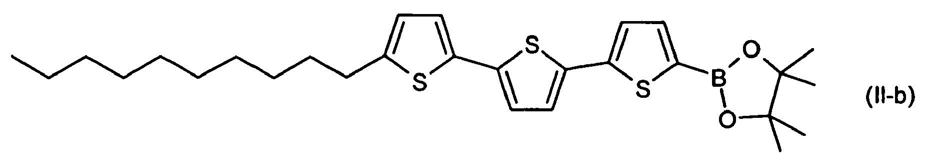

2-(5"-Decyl-2,2':5',2"-terthien-5-yl)-4,4,5,5-tetramethyl-1,3,2-dioxaborolan, 2.4 ml einer 2.5 M Butyllithiumlösung in Hexan, 5-Decyl-2,2':5',2"-terthiophen (2.33 g, 6 mmol) und 2-Isopropoxy-4,4,5,5-tetramethyl-1,3,2-dioxaborolan (1.4 ml, 7 mmol) werden wie in Beispiel 1 beschrieben in 80 ml wasserfreiem THF umgesetzt und das entstehende Produkt isoliert. Ausbeute: 3.02 g (98 % der Theorie) olivgrünes, festes Produkt (II-b).

### Analysendaten:

EI MS Analyse: M^{•+} ca. 90%, m/e = 514.
¹H NMR (CDCl₃, TMS/ppm): 0.880 (3H, t, J=6.9 Hz), 1.20-1.45 (overlapped peaks, 14 H), 1.351 (s, 12 H), 1.678 (2H, m, J=7.5 Hz), 2.787 (2H, t, J=7.6 Hz), 6.679 (1H, d, J=3.4 Hz), 6.988 (2H, dd, J1=3.9 Hz, J2=3.9 Hz), 7.111 (1H, d, J=3.9 Hz), 7.209 (1H, d, J=3.9 Hz), 7.516 ( 1 H, d, J=3.4 Hz).

### Beispiel 3: Herstellung von 5-Decyl-5"-[4-(5"-decyl-2,2':5',2"-terthien-5-yl)phenyl]-2,2':5',2"-terthiophen (I-a-1)

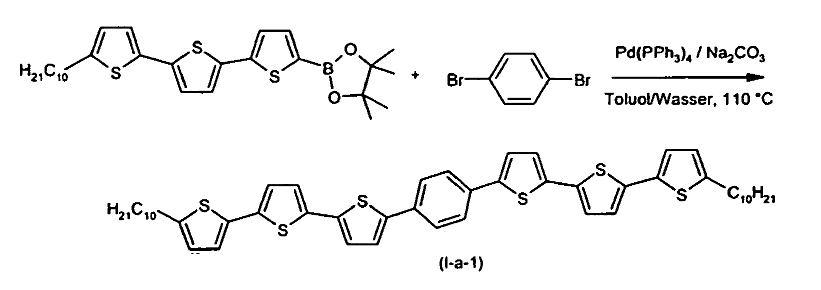

1,4-Dibrombenzol (142 mg, 0.6 mmol) wurde vorgelegt und unter Schutzgas Tetrakis(triphenylphosphin)palladium Pd(PPh₃)₄ (46 mg, 0.04 mmol) dazugegeben. Es wurde eine Lösung von 2-(5"-Decyl-2,2':5',2"-terthien-5-yl)-4,4,5,5-tetramethyl-1,3,2-dioxaborolan (II-b) (865 mg, 1.7 mmol) in 20 ml wasserfreiem Toluol und 5 ml einer 2 M wässrigen Na₂CO₃-Lösung vorbereitet und mit Stickstoff desoxidiert. Beide Lösungen wurden mit Spritzen zum Reaktionsansatz gegeben und das Reaktionsgemisch anschließend für 20 Stunden unter Rückfluss erhitzt. Das Reaktionsgemisch wurde nach Abkühlen in ein Gemisch aus 200 ml Wasser, 80 ml 1 M HCl und 300 ml Toluol gegeben. Nach dreimaligem Ausschütteln mit je 100 ml Wasser wurde die organische Phase über einen G4 Glasfilter filtriert und der Filterrückstand getrocknet. Ausbeute: 404 mg (79 % der Theorie) orangefarbenes Pulver.

### Analysendaten:

EI MS Analyse: M^{•+} 99+%, m/e = 850.3
EI MS Analyse (nach Aufreinigung durch Sublimation bei 0,25 mBar, 320°C)): M^{•+} 100%, m/e = 850.3.
Schmelzverhalten (°C): K 310 I (K = kristallin, I = isotrop flüssig).

Das Schmelzverhalten wurden bestimmt mit einem DSC (Differential Scanning Calorimeter) Mettler TA-4000 Thermosystem, Scanning rate 10K/min.

## Patentansprüche

1. Verfahren zur Herstellung von Verbindungen der allgemeinen Formel (I) 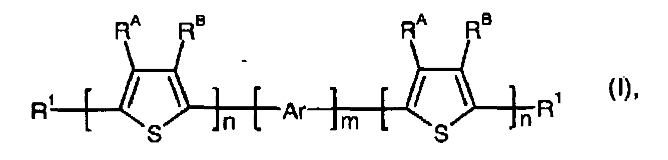 insbesondere nach dem Prinzip der Suzuki-Kupplung,
worin
m für eine ganze Zahl von 1 bis 5 steht,
Ar für gegebenenfalls substituiertes 1,4-Phenylen und/oder 2,7-Fluorenylen steht,
R¹ für H oder eine gegebenenfalls subsituierte lineare oder verzweigte C₁-C₂₀ -Alkylgruppe, eine gegebenenfalls durch ein(e) oder mehrere O- oder S-Atome, Silylen-, Phosphonoyl- oder Phosphorylgruppen unterbrochene lineare C₁-C₂₀-Alkylgruppe oder eine C₃-C₂₀-Alkenylgruppe steht.
R^{A},R^{B} unabhängig voneinander jeweils für H oder eine gegebenenfalls substituierte und/oder gegebenenfalls mit bis zu 10 Sauerstoffatomen unterbrochene lineare oder verzweigte C₁-C₂₀-Alkylgruppe, eine gegebenenfalls substituierte C₁-C₂₀-Alkoxygruppe oder gemeinsam für eine gegebenenfalls substituierte C₁-C₆-Dioxyalkylengruppe stehen und
n für 2, 3 oder 4 steht,
**dadurch gekennzeichnet, dass** eine Verbindung der allgemeinen Formel (II) 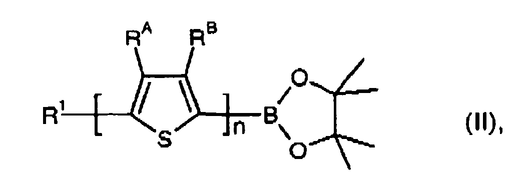 worin
n, R¹, R^{A} und R^{B} die oben für die allgemeine Formel (I) genannte Bedeutung haben,
mit einer Verbindung der allgemeinen Formel (III) 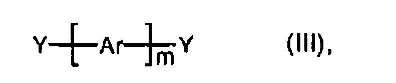 worin
m und Ar die für die allgemeine Formel (1) genannte Bedeutung haben und
Y für einen Rest Cl, Br, I oder O-SO₂-R^{C} steht, wobei R^{C} für einen Phenyl-, Tolyl-, Methyl- oder Trifluormethylrest steht,
gegebenenfalls in Anwesenheit von Katalysatoren, gegebenenfalls in Lösung und gegebenenfalls bei erhöhter Temperatur umgesetzt wird.

2. Verfahren gemäß Anspruch 1 zur Herstellung von Verbindungen der allgemeinen Formel (I-a) 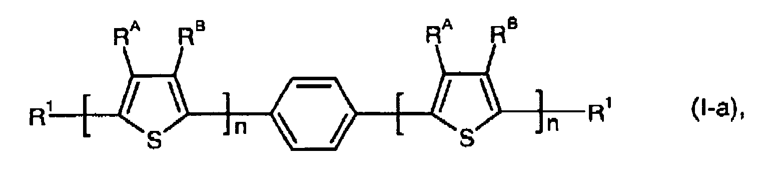 worin
n, R¹, R^{A} und R^{B} die in Anspruch 1 genannte Bedeutung haben,
**dadurch gekennzeichnet, dass** eine Verbindung der allgemeinen Formel (II) mit einer Verbindung der allgemeinen Formel (III-a) 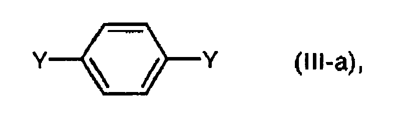 worin
Y die in Anspruch 1 genannte Bedeutung hat,
umgesetzt wird.

3. Verfahren gemäß Anspruch 1 oder 2, **dadurch gekennzeichnet, dass** R^{A} und R^{B} für H stehen.

4. Verfahren gemäß wenigstens einem der Ansprüche 1 bis 3, **dadurch gekennzeichnet, dass** die Umsetzung in Gegenwart wenigstens eines Katalysators enthaltend ein Metall der VIII. Nebengruppe des Periodensystems der Element erfolgt.

5. Verfahren gemäß wenigstens einem der Ansprüche 1 bis 4, **dadurch gekennzeichnet, dass** die Umsetzung in Gegenwart wenigstens einer Base erfolgt.

6. Verfahren gemäß wenigstens einem der Ansprüche 1 bis 5, **dadurch gekennzeichnet, dass** die Umsetzung in Gegenwart wenigstens eines organischen Lösungsmittels erfolgt.

## Claims

1. Process for the preparation of compounds of the general formula (I), 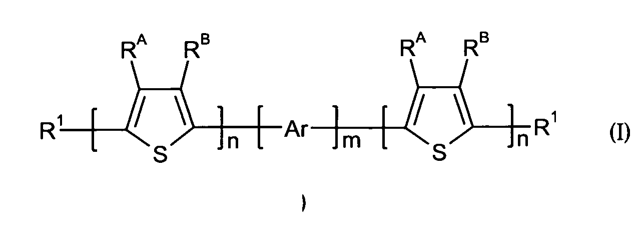 in particular in accordance with the principle of Suzuki coupling,
wherein
m represents an integer from 1 to 5,
Ar represents optionally substituted 1,4-phenylene and/or 2,7-fluorenylene,
R¹ represents H or an optionally substituted linear or branched C₁-C₂₀-alkyl group, a linear C₁-C₂₀-alkyl group which is optionally interrupted by one or more O or S atoms or silylene, phosphonoyl or phosphoryl groups, or a C₃-C₂₀-alkenyl group,
R^{A}, R^{B} independently of each other each represent H or a linear or branched C₁-C₂₀-alkyl group which is optionally substituted and/or optionally interrupted by up to 10 oxygen atoms, an optionally substituted C₁-C₂₀-alkoxy group, or together represent an optionally substituted C₁-C₆-dioxyalkylene group and
n represents 2, 3 or 4,
**characterized in that** a compound of the general formula (II) 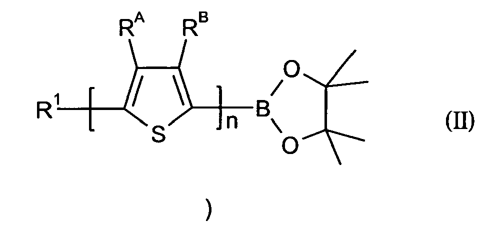 wherein
n, R¹, R^{A} and R^{B} have the meaning given above for the general formula (I) is reacted with a compound of the general formula (III) 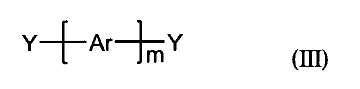 wherein
m and Ar have the meaning given for the general formula (I) and
Y represents a radical Cl, Br, I or O-SO₂-R^{C}, wherein R^{C} represents a phenyl, tolyl, methyl or trifluoromethyl radical,
optionally in the presence of catalysts, optionally in solution and optionally at elevated temperature.

2. Process according to claim 1 for the preparation of compounds of the general formula (I-a) 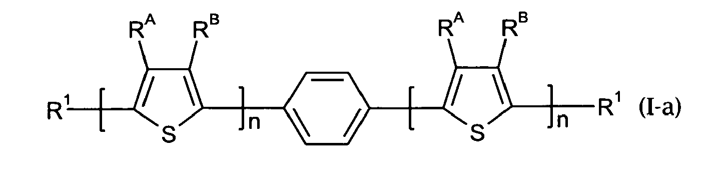 wherein
n, R¹, R^{A} and R^{B} have the meaning given in claim 1,
**characterized in that** a compound of the general formula (II) is reacted with a compound of the general formula (III-a) 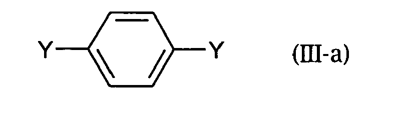 wherein
Y has the meaning given in claim 1.

3. Process according to claim 1 or 2, **characterized in that** R^{A} and R^{B} represent H.

4. Process according to at least one of claims 1 to 3, **characterized in that** the reaction is carried out in the presence of at least one catalyst containing a metal of sub-group VIII of the periodic table of the elements.

5. Process according to at least one of claims 1 to 4, **characterized in that** the reaction is carried out in the presence of at least one base.

6. Process according to at least one of claims 1 to 5, **characterized in that** the reaction is carried out in the presence of at least one organic solvent.

## Revendications

1. Procédé pour la préparation de composés de la formule générale (I) 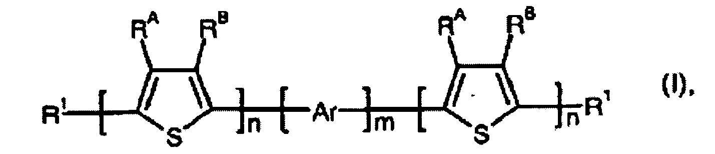 en particulier d'après le principe du couplage de Suzuki,
dans laquelle
m représente un nombre entier de 1 à 5,
Ar représente un 1,4-phénylène, substitué le cas échéant et/ou un 2,7-fluorénylène,
R¹ représente un H ou un groupe alkyle linéaire ou ramifié en C₁ à C₂₀, substitué le cas échéant, un groupe alkyle linéaire en C₁ à C₂₀ interrompu le cas échéant par un ou plusieurs atomes de O ou de S, ou par un ou plusieurs groupes silylènes, phosphonoyles ou phosphoryles, ou un groupe alcényle en C₃ à C₂₀,
R^{A}, R^{B} représentent, indépendamment l'un de l'autre, respectivement un H ou un groupe alkyle linéaire ou ramifié en C₁ à C₂₀, substitué le cas échéant et/ou interrompu le cas échéant par jusqu'à 10 atomes d'oxygène, un groupe alkoxyle en C₁ à C₂₀, substitué le cas échéant ou, ensemble, un groupe dioxyalkylène en C₁ à C₆, substitué le cas échéant et
n représente 2, 3 ou 4,
**caractérisé en ce qu'**un composé de la formule générale (II) 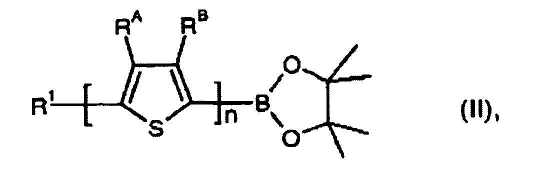 dans laquelle
n, R¹, R^{A} et R^{B} ont la signification donnée ci-dessus pour la formule générale (I),
réagit avec un composé de la formule générale (III) 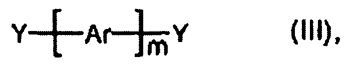 dans laquelle
m et Ar ont les significations données pour la formule générale (I), et
Y représente un radical Cl, Br, I, ou O-SO₂-R^{C}, R^{C} représentant un radical phényle, toluyle, méthyle ou trifluorométhyle,
le cas échéant en présence de catalyseurs, le cas échéant en solution, et le cas échéant à température accrue.

2. Procédé selon la revendication 1 pour la préparation de composés de la formule générale (I-a) 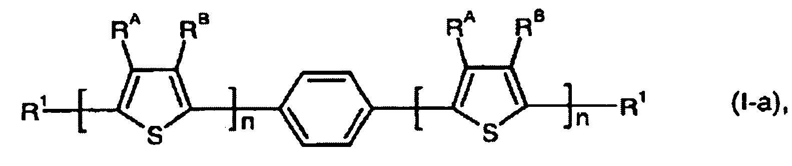 dans laquelle
n, R¹, R^{A} et R^{B} ont les significations données à la revendication 1,
**caractérisé en ce qu'**un composé de la formule générale (II) réagit avec un composé de la formule générale (III-a) 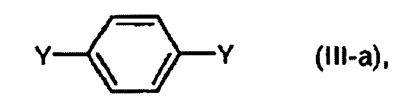 dans laquelle
Y a la signification donnée à la revendication 1.

3. Procédé selon la revendication 1 ou 2, **caractérisé en ce que** R^{A} et R^{B} représentent des H.

4. Procédé selon l'une au moins des revendications 1 à 3, **caractérisé en ce que** la réaction s'effectue en présence d'au moins un catalyseur contenant un métal de transition du VIIIe sous-groupe du tableau périodique des éléments.

5. Procédé selon l'une au moins des revendications 1 à 4, **caractérisé en ce que** la réaction s'effectue en présence d'au moins une base.

6. Procédé selon l'une au moins des revendications 1 à 5, **caractérisé en ce que** la réaction s'effectue en présence d'au moins un solvant organique.
